# EUROPÄISCHE PATENTSCHRIFT

(11) **EP 0 530 229 B1**
(45) Veröffentlichungstag und Bekanntmachung des Hinweises auf die Patenterteilung: **28.06.1995**
(21) Anmeldenummer: 91909057.1
(22) Anmeldetag: 10.05.1991
(51) Int. Cl.: A61K 7/13

(54) **OXIDATIONSFÄRBEMITTEL FÜR KERATINFASERN**
OXIDATION COLORANTS FOR KERATIN FIBRES
COLORANTS D'OXYDATION POUR FIBRES DE KERATINE

(30) Priorität: 19.05.1990 DE 4016177
(43) Veröffentlichungstag der Anmeldung: 10.03.1993
(73) Patentinhaber: Henkel Kommanditgesellschaft auf Aktien, 40191 Düsseldorf (DE)
(72) Erfinder: KONRAD, Günther, D-4010 Hilden (DE); MATZIK, Iduna, D-4006 Erkrath 2 (DE); LIESKE, Edgar, D-4000 Düsseldorf (DE)
(86) Internationale Anmeldenummer: EP9100874
(87) Internationale Veröffentlichungsnummer: WO9117739

(56) Entgegenhaltungen:
- DE-A- 1 916 139
- GB-A- 2 033 392
- US-A- 4 013 404
- Journal of Chemical Society, C, Band 15, 1970, (Newcastle upon Tyne, GB), F.Binns et al.: "Studies related to the chemistry of melanins. Part XIII. Studies on the structure of dopamine-melanin", Seiten 2063-2075, siehe Seite 2067, Spalte 1, Zeilen 26-27

## Beschreibung

Die Erfindung betrifft die Verwendung von Indolin-Derivaten, insbesondere von 5,6-Dihydroxyindolinen als Oxidationsfarbstoffvorprodukt Zur Herstellung von Oxidationsfärbemitteln für Keratinfasern, insbesondere für menschliches Haar.

Die natürlichen Haarfarbstoffe, die sogenannten Melanine, werden im Verlaufe ihrer Biosynthese durch oxidative Polymerisation von 5,6-Dihydroxyindol gebildet. Es hat daher in der Vergangenheit zahlreiche Versuche gegeben, das 5,6-Dihydroxyindol als reaktives Farbstoffvorprodukt in der Haarfärberei zu verwenden. Leider ist das 5,6-Dihydroxyindol sowohl in freier Form als auch in Form seiner Salze in wäßriger Lösung äußerst instabil und bildet in Gegenwart von Luftsauerstoff sehr rasch unlösliche, gefärbte Oxidations- und Polymerisationsprodukte, die selbst nicht mehr am Haar fixiert werden können. Daher haben alle Versuche, das 5,6-Dihydroxyindol selbst oder dessen Salze in Färbezubereitungen einzusetzen, zu unbefriedigenden und kommerziell nicht verwendbaren Haarfärbemitteln geführt.

Es wurde nunmehr eine neue und überraschende Möglichkeit gefunden, natürliche Haarfärbungen mit Melanin-Farbstoffen über ein "in situ" gebildetes, 5,6-Dihydroxyindol und ohne die für das 5,6-Dihydroxyindol bekannten Stabilitätsprobleme zu erzeugen.

Gegenstand der Erfindung ist die Verwendung von Indolinen der Formel I 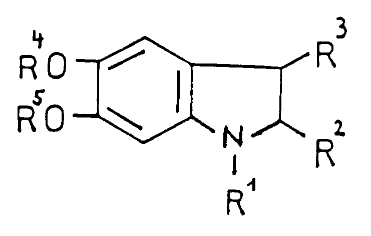
worin R¹, R², R³, R⁴ und R⁵ unabhängig voneinander Wasserstoff oder Alkylgruppen mit 1 - 4 C-Atomen oder R⁴ und R⁵ auch gemeinsam mit den Sauerstoffatomen, an die sie gebunden sind, eine Alkylendioxygruppe mit 1 - 4 C-Atomen darstellen, oder deren Salze als Oxidationsfarbstoffvorprodukte zur Erzeugung von Oxidationsfärbungen.

Bevorzugt geeignet ist 5,6-Dihydroxyindolin, also das Indolderivat der Formel I, worin R¹, R², R³, R⁴ und R⁵ Wasserstoff ist, da dieses bei der Oxidation 5,6-Dihydroxyindol und weiterhin Melanin-Farbstoff bildet. Es ist aber bekannt, daß auch Derivate des 5,6-Dihydroxyindols unter Oxidationsbedingungen dem Melanin ähnliche Farbstoffe bilden. Entsprechend eignen sich die alkylsubstituierten Indoline Formel I, bevorzugt solche, bei welchen eine der Gruppen R¹, R² und R³ eine Methylgruppe ist und die übrigen Wasserstoff sind, als Oxidationsfarbstoffvorprodukte zur Herstellung lagerstabiler Färbezubereitungen.

Wegen der chemischen Analogie der aus den erfindungsgemäß zu verwendenden Indoline entstehenden Melanin-Farbstoffe zum natürlichen Melanin-Farbstoff kann ein günstiges toxikologisches und dermatologisches Verhalten der damit hergestellten Färbezubereitungen erwartet werden. Vor allem aber werden besonders natürliche Farbnuancen im Bereich von mittelblond bis mittelbraun mit hoher Echtheit gegenüber Licht, mechanischer Reibung, chemischer Behandlung, z.B. mit reduzierender Kaltwellzubereitung und gegenüber dem Waschen mit Tensidlösungen erzielt.

Das 5,6-Dimethoxyindolin und das 5,6-Dihydroxyindolin sind literaturbekannt, ihre Herstellung ist z.B. in J. Chem. Soc. (C), 1967, Seiten 1424 bis 1427 beschrieben. In analoger Weise lassen sich die alkylsubstituierten Indoline der Formel I aus den entsprechend substituierten 5,6-Dihydroxyindolen oder Alkoxyindolen durch katalytische Hydrierung herstellen. Ein anderes Verfahren zur Herstellung von 5,6-Dihydroxyindolinen aus 5,6-Dimethoxyindolen durch Reduktion mit Natrium-cyanoborhydrid und Abspaltung der Methoxygruppen in konzentrierter Salzsäure ist im Journal of Medicinal Chemistry, 1978, Vol 21, No. 6, Seite 553 beschrieben.

Die erfindungsgemäß zu verwendenden Indoline der Formel I werden bevorzugt als einzige Oxidationsfarbstoffvorprodukte eingesetzt. Sie können dabei in freier Form oder in Form ihrer Salze, bevorzugt als Hydrochloride, Hydrobromide, Sulfate, Phosphate, Acetate, Propionate, Lactate oder Citrate eingesetzt werden.

Es können aber auch zur Modifikation der Farbnuancen andere bekannte Oxidationsfarbstoffvorprodukte und gegebenenfalls auch bekannte direktziehende Farbstoffe gemeinsam mit den Indolinen der Formel I eingesetzt werden.

Insbesondere wurde gefunden, daß die Indoline der Formel I sowie deren Salze sich auch gut zur Verwendung als Kupplerkomponenten in Oxidationshaarfärbemitteln mit einem Gehalt an üblichen Entwicklerverbindungen eignen.

Ein weiterer Erfindungsgegenstand sind daher Haarfärbemittel, enthaltend Oxidationsfarbstoffvorprodukte in einem Träger, dadurch gekennzeichnet, daß als Kupplerkomponenten Indoline der Formel I oder deren Salze sowie die für Oxidationshaarfärbemittel üblichen Entwicklerverbindungen enthalten sind. Die Indoline der Formel I modifizieren dabei die mit den Entwicklerverbindungen allein durch Selbstkupplung erzielbaren Färbungen und führen zu intensiven, brillanten dunkelbraunen bis blauschwarzen Nuancen.

Solche Haarfärbemittel enthalten bevorzugt einen schwach basischen Träger.

Als übliche Entwicklerkomponenten können alle hierfür bekannten Verbindungen eingesetzt werden. Bevorzugt sind dabei solche vom Typ der aromatischen oder heterocyclischen Aminoverbindungen. Solche geeigneten Entwicklerverbindungen sind z.B. p-Phenylendiamin, p-Toluylendiamin, 2-Chlor-p-Phenylendiamin, N-Methyl-p-Phenylendiamin, N,N-Diethyl-p-phenylendiamin, N-(β-Hydroxyethyl)-p-phenylendiamin, N,N-Dimethyl-p-phenylendiamin, N,N-Bis-(β-hydroxyethyl)-p-Phenylendiamin, 2,5-Diaminobenzylalkohol und andere Verbindungen der genannten Art, die weiterhin eine oder mehrere NH₂-Gruppen, NHR-Gruppen oder NR₂-Gruppen enthalten können, worin R Alkylgruppen mit 1 bis 4 C-Atomen oder Hydroxyalkylgruppen mit 2 bis 4 C-Atomen sind sowie p-Aminophenole, 1-Amino-4-naphthol oder Tetraaminopyrimidine, z.B. 2,4,5,6-Tetraaminopyrimidin oder 2-Dimethylamino-4,5,6-triaminopyrimidin, Diaminopyrimidine oder 1-Phenyl-3-carboxamido-4-amino-pyrazolon-5.

Wenn die Indoline der Formel I als Kuppler zur Modifizierung der Nuance der genannten Entwicklerverbindungen eingesetzt werden, so werden sie in Mengen von 0,1 bis 10 Millimol pro 100 g des Haarfärbemittels verwendet. Sie werden dabei üblicherweise in etwa molaren Mengen bezogen auf die verwendeten Entwicklersubstanzen eingesetzt. Wenn sich auch der molare Einsatz als zweckmäßig erwiesen hat, so ist auch ein Überschuß einzelner Oxidationsfarbstoffvorprodukte nicht nachteilig.

Es ist nicht erforderlich, daß die Indole der Formel I einheitliche chemische Verbindungen darstellen, vielmehr können diese auch Gemische der erfindungsgemäß zu verwendenden Indole der Formel I oder deren Salze sein.

Zur Herstellung von Oxidationsfärbemitteln werden die Oxidationsfarbstoffvorprodukte in einem geeigneten Träger eingearbeitet. Solche Träger sind z.B. Cremes, Emulsionen, Gele oder auch tensidhaltige, schäumende Lösungen (Shampoos), Schaumaerosole oder sonstige Zubereitungen, die für die Anwendung auf dem Haar geeignet sind. Solche Träger enthalten Konfektionierungs- und Färbehilfsmittel, welche die Stabilität der Zubereitungen erhöhen und das Ergebnis der Färbung verbessern. Solche Zusätze sind in erster Linie oberflächenaktive Stoffe, z.B.
- Seifen, insbesondere die Alkali- oder Alkanolaminseifen von linearen C₁₂-C₁₈-Fettsäuren, insbesondere von Ölsäure,
- anionische Tenside, z.B. Fettalkoholsulfate und Fettalkoholpolyglycolethersulfate, Alkansulfonate, alpha-Olefinsulfonate oder Ölsäuresulfonate, bevorzugt in Form der Alkali-, Ammonium- oder Alkanolammoniumsalze
- kationische Tenside, z.B. Alkyl (C₁₂-C₁₈)-trimethyl-ammoniumchloride, Alkyl(C₁₂-C₁₈)-dimethyl-benzyl-ammoniumsalze, Cetylpyridiniumchlorid, 2-Hydroxydodecyl-hydroxyethyl-dimethylammoniumchlorid
- zwitterionische Tenside, wie z.B. Alkyl-(C₁₂-C₁₈)-dimethyl-ammonium-glycinat, Kokosacylaminopropyl-dimethyl-ammonium-glycinat, oder Imidazoliniumbetaine
- amphotere Tenside, wie z.B. N-Dodecylaminoessigsäure, N-Cetylaminopropionsäure, gamma-Laurylamino-buttersäure und
- nichtionische Tenside, insbesondere Anlagerungsprodukte von 5 - 30 Mol Ethylenoxid an Fettalkohole, an Alkylphenole, an Fettsäuren, an Fettsäurealkanolamide, an Fettsäurepartialglyceride, an Fettsäure-sorbitanpartialester oder an Fettsäure-methylglucosid-partialester, ferner Alkylglucoside, Aminoxide und Fettsäure-polyglycerinester.

Weitere Konfektionierungshilfsmittel sind die
- wasserlöslichen, verdickenden Polymeren (Hydrokolloide), z.B. Celluloseether wie Carboxymethylcellulose, Hydroxyethylcellulose, Methylcellulose, Methyl-hydroxypropylcellulose, Stärke und Stärkeether, Pflanzengumme, Guar-Gum, Agar-Agar, Alginate, Xanthan-Gum oder synthetische wasserlösliche Polymere.
- Antioxidantien, z.B. Ascorbinsäure, Na₂SO₃,
- Puffersubstanzen, z.B. Ammoniumchlorid und Ammoniumsulfate,
- Komplexbildner, z.B. 1-Hydroxyethan-1,1-diphosphonsäure, Nitrilotriessigsäure oder Ethylendiamintetraessigsäure oder deren Salze,
- Haarkosmetische Hilfsmittel, z.B. wasserlösliche kationische Polymere, Proteinderivate, Glucose, D-Panthenol, Cholesterin, Vitamine oder Pflanzenextrakte,
- Egalisierhilfsmittel, z.B. Urazol, Hexahydropyrimidin-2-on, Imidazol, 1,2,4-Triazol oder Jodide, z.B. Natrium- oder Kaliumiodid

Die Anwendung der erfindungsgemäßen Haarfärbemittel kann im schwach sauren, neutralen oder schwach alkalischen pH-Bereich erfolgen.

Eine bevorzugte Ausführungsform der Erfindung sind Haarfärbemittel mit einem Gehalt an Oxidationsfarbstoffvorprodukten in einem Träger, die als Oxidationsfarbstoffvorprodukte Indoline der Formel I oder deren Salze in einer Menge von 0,1 - 20 Millimol pro 100 g des Haarfärbemittels und als Träger ein Gel mit einem Gehalt von 1 - 20 Gew.-% einer Seife oder eine Öl-in-Wasser-Emulsion mit einem Gehalt von 1 - 25 Gew.-% einer Fettkomponente und 0,5 - 30 Gew.-% eines Emulgiermittels aus der Gruppe der anionischen, nichtionischen, kationischen, zwitterionischen oder ampholytischen Tenside enthalten.

Als neutrale oder schwach saure Träger für die Färbezubereitung eignen sich bevorzugt Öl-in-Wasser-Creme-Emulsionen mit einem Gehalt von Fettalkoholen mit 12 - 22 C-Atomen, bevorzugt von Cetyl- und Stearylalkohol als Fettkomponente und nichtionogenen zwitterionischen oder kationischen Emulgatoren, bevorzugt Anlagerungsprodukten von 10 - 30 Mol Ethylenoxid an Cetyl- und Stearylalkohol, die gegebenenfalls durch Zusatz von Citronensäure oder anderer schwacher Säuren auf einen pH-Wert von 2,5 - 4 eingestellt werden.

In diese Emulsion wird das erfindungsgemäß zu verwendende Indolin der Formel I in einer Menge von 2 - 20 Millimol pro 100 g eingearbeitet.

Als schwach basische Träger für die Färbezubereitung eignen sich bevorzugt Gele oder Öl-in-Wasser-Emulsionen. Geeignete Gele enthalten als oberflächenaktive Stoffe 1 - 20 Gew.-% einer Seife, bevorzugt Ammoniumoleat, bevorzugt zusätzlich 1 - 10 Gew.-% eines nichtionischen Emulgators und 5 - 20 Gew.-% eines Fettalkohols mit 12 - 22 C-Atomen als Fettkomponente. Geeignete Öl-in-Wasser-Emulsionen enthalten 1 - 25 Gew.-% einer Fettkomponente, bevorzugt eines Fettalkohols mit 12 - 22 C-Atomen, und 0,5 - 30 Gew.-% eines Emulgiermittels, bevorzugt 1 - 20 Gew.-% eines anionischen, nichtionischen, zwitterionischen oder ampholytischen Tensids.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird.

Die Oxidation kann entweder unmittelbar vor der Anwendung der Färbezubereitung durch Vermischen mit einem Oxidationsmittel eingeleitet werden oder erst auf dem Haar erfolgen. Im ersten Falle wird die Färbezubereitung mit einem Oxidationsmittel, bevorzugt mit Wasserstoffperoxid-Lösungen oder mit Wasserstoffperoxid-Anlagerungsprodukten an Harnstoff, Melamin oder Natriumborat vermischt und nach kurzer Einwirkungszeit auf das Haar aufgetragen, wobei der Oxidationsprozess und die Entwicklung des Farbstoffes dann während der Einwirkungszeit auf dem Haar vollendet wird. Diese Anwendung ist recht einfach durchzuführen und eignet sich besonders für die Heimanwendung.

Tiefere und brillantere Färbungen werden erzielt, wenn man zunächst die Färbezubereitung auf das Haar aufträgt, 10 - 30 Minuten einwirken läßt und dann erst eine Oxidationsmittelzubereitung auf das Haar aufträgt. Als Oxidationsmittel eignen sich bevorzugt Wasserstoffperoxidlösungen oder Dispersionen von Kalium- oder Ammoniumperoxydisulfat oder wasserlösliche Perjodate. Diese Dispersionen können ähnlich zusammengesetzt sein wie die vorher für neutrale oder schwach saure Färbezubereitungen beschriebenen Träger in Form einer Creme-Emulsion. Die separate Aufbringung des Oxidationsmittels auf das Haar erfordert mehr Sorgfalt und Übung und ist daher bevorzugt für die gewerbliche Anwendung beim Friseur geeignet.

Nach einer Einwirkungszeit von 15 - 30 Minuten werden überschüssiges Färbe- und Oxidationsmittel aus dem Haar ausgewaschen. Hierzu verwendet man bevorzugt ein handelsübliches anionisches Shampoo. Wenn die Färbezusammensetzung bereits einen ausreichenden Tensidgehalt aufweist, kann man auch mit Wasser auswaschen.

Die folgenden Beispiele sollen den Erfindungsgegenstand näher erläutern ohne ihn hierauf zu beschränken.

### Beispiele

### 1. Färbeverfahren schwach saurer, Oxidation auf dem Haar

Es wurde eine Färbecreme-Emulsion folgender Zusammensetzung hergestellt:

| | |
|---|---|
| Cetyl-/Stearylalkohol (30:70) | 6 g |
| Kokosfettalkohol (C₁₂-C₁₈) | 2 g |
| Cetyl-/Stearylalkohol-polyglycoleter (20 EO) | 2 g |
| 5,6-Dihydroxyindolin-hydrobromid | 1 g |
| Wasser | 89 g |

Es wurde eine Oxidationsmittel-Dispersion der folgenden Zusammensetzung hergestellt:

| | |
|---|---|
| Cetyl-/Stearylalkohol (30:70) | 6 g |
| Kokosfettalkohol (C₁₂-C₁₈) | 2 g |
| Cetyl-/Stearylalkohol-polyglycolether (20 EO) | 2 g |
| Ammoniumperoxydisulfat | 5 g |
| Wasser | 85 g |

### Haaranfärbung

Die Färbecreme wurde auf Haarsträhnen von 15 cm Länge und ca. 2 g Gewicht von unvorbehandeltem, standardisiertem, zu 80 % ergrautem Menschenhaar aufgetragen und 20 Minuten bei 25 °C einwirken gelassen.

Ohne Zwischenspülung wurde dann die Oxidationsmitteldispersion auf die gleichen Haarsträhnen aufgetragen und 20 Minuten einwirken gelassen. Danach wurden die Strähnen mit einem üblichen Shampoo gewaschen, mit Wasser gespült und getrocknet.

Es wurde ein mittelbrauner Farbton und eine sehr gute Grauabdeckung der Prüfsträhne erzielt. Die Lichtechtheit, Reibechtheit und Beständigkeit gegenüber einer Dauerwellbehandlung waren sehr gut.

### 2. Färbeverfahren schwach alkalisch, Oxidation vor dem Auftragen auf das Haar

Es wurde eine Färbecreme-Emulsion folgender Zusammensetzung hergestellt:

| | |
|---|---|
| Lauryl-/Myristylalkohol (70:30) | 10 g |
| Fettalkohol (C_{12/14})-ethersulfat (2 EO), Natriumsalz, 28%ige Lösung in Wasser | 25 g |
| Wasser | 60 g |
| Na₂SO₃ | 1 g |
| Ammoniumsulfat | 1 g |
| 5,6 Dihydroxyindolin-hydrobromid konzentrierte Ammoniak-Lösung bis pH = 9,5 | 1 g |
| Wasser | ad 100 g |

Die Bestandteile wurden der Reihe nach miteinander vermischt. Nach Zugabe des 5,6-Dihydroxyindolins und des Ammoniumsulfats (Puffer) wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Es resultierte eine brillante, farbintensive Nuance im Dunkelbraunbereich.

Die Ausfärbung zeichnete sich durch sehr gute Echtheitseigenschaften aus (z.B. Lichtechtheit, Reibechtheit, Kaltwellechtheit, Waschechtheit).

### 3. Einsatz als Kupplerkomponente gemeinsam mit bekannten Entwicklerkomponenten.

Es wurden erfindungsgemäße Haarfärbemittel in Form einer Haarfärbe-Cremeemulsion der folgenden Zusammensetzung hergestellt:

| | |
|---|---|
| Fettalkohol C₁₂-C₁₈ | 10 g |
| Fettalkohol(C₁₂-C₁₄)-ethersulfat(2EO) Natriumsalz, 28%ig in Wasser | 25 g |
| Wasser | 60 g |
| Na₂SO₃ | 1 g |
| (NH₄)₂SO₄ | 1 g |
| 5,6-Dihydroxyindolin-hydrobromid | 1,74 g |
| Entwickler (Komponente E) konzentrierte Ammoniak-Lösung bis pH = 9,5 | 7,5 mMol |
| Wasser | ad 100 g |

Die Herstellung der Cremeemulsion, die oxidative Entwicklung und die Ausfärbung auf Haarsträhnen erfolgte analog Beispiel 2.

Als Entwickler (Komponente E) wurden nacheinander die folgenden Verbindungen eingesetzt:
- E1:: p-Toluylendiamin
- E2:: 2-Chlor-p-phenylendiamin
- E3:: N-Methyl-p-phenylendiamin
- E4:: N,N-Diethyl-p-phenylendiamin
- E5:: N-(β-Hydroxyethyl)-p-phenylendiamin
- E6:: N,N-Dimethyl-p-phenylendiamin
- E7:: N,N-Bis-(β-hydroxyethyl)-p-phenylendiamin
- E8:: 2,5-Diaminobenzylalkohol
- E9:: p-Aminophenol
- E10:: 1-Amino-4-naphthol
- E11:: 2,4,5,6-Tetraaminopyrimidin

Die Ergebnisse der Haarfärbeversuche sind der folgenden Tabelle zu entnehmen:

| Haarfärbemittel | Entwickler Komponente E | erhaltende Farbnuance |
|---|---|---|
| 3.1 | E1 | schwarz |
| 3.2 | E2 | dunkelviolett |
| 3.3 | E3 | schwarzviolett |
| 3.4 | E4 | schwarzblau |
| 3.5 | E5 | schwarzblau |
| 3.6 | E6 | blauschwarz |
| 3.7 | E7 | blauschwarz |
| 3.8 | E8 | dunkelviolett |
| 3.9 | E9 | dunkelbraun |
| 3.10 | E10 | dunkelbraun |
| 3.11 | E11 | braunschwarz |

## Patentansprüche

1. Verwendung von Indolinen der Formel I 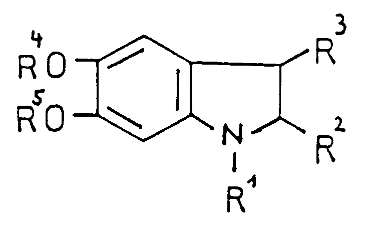 worin R¹, R², R³, R⁴ und R⁵ unabhängig voneinander Wasserstoff oder Alkylgruppen mit 1 - 4 C-Atomen oder R⁴ und R⁵ gemeinsam mit den Sauerstoffatomen, an die sie gebunden sind, eine Alkylendioxygruppe mit 1 - 4 C-Atomen darstellen, oder deren Salze als Oxidationsfarbstoffvorprodukte zur Erzeugung von Oxidationsfärbungen.

2. Verwendung nach Patentanspruch 1, dadurch gekennzeichnet, daß in der Formel I die Gruppen R¹, R², R³, R⁴ und R⁵ Wasserstoff sind oder gegebenenfalls eine der Gruppen R¹, R² und R³ eine Methylgruppe ist und die übrigen Wasserstoff sind.

3. Verwendung nach Anspruch 1 oder 2, dadurch gekennzeichnet, daß die Indoline der Formel I oder deren Salze als Kupplerkomponenten in Oxidationshaarfärbemitteln mit einem Gehalt an üblichen Entwicklerverbindungen enthalten sind.

4. Haarfärbemittel, enthaltend Oxidationsfarbstoffvorprodukte in einem Träger, dadurch gekennzeichnet, daß als Oxidationsfarbstoffvorprodukte Indoline der Formel I gemäß Anspruch 1 oder 2 oder deren Salze in einer Menge von 0,1 - 20 Millimol pro 100 g des Haarfärbemittels und als Träger ein Gel mit einem Gehalt von 1 - 20 Gew.-% einer Seife oder eine Öl-in-Wasser-Emulsion mit einem Gehalt von 1 - 25 Gew.-% einer Fettkomponente und 0,5 - 30 Gew.-% eines Emulgiermittels aus der Gruppe der anionischen, nichtionischen, kationischen oder ampholytischen Tenside enthalten sind.

5. Haarfärbemittel nach Anspruch 4, dadurch gekennzeichnet, daß als Kupplerkomponenten Indoline der Formel I gemäß Anspruch 1 oder 2 oder deren Salze sowie die für Oxidationshaarfärbemittel üblichen Entwicklerverbindungen enthalten sind.

## Claims

1. The use of indolines corresponding to formula I 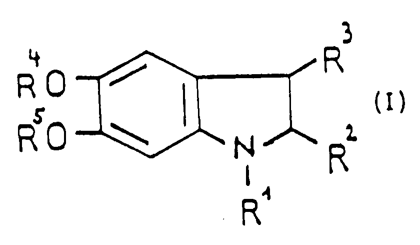 in which R¹, R², R³, R⁴ and R⁵ independently of one another represent hydrogen or C₁₋₄ alkyl groups or R⁴ and R⁵ together with the oxygen atoms to which they are attached may represent an alkylenedioxy group containing 1 to 4 carbon atoms,
or salts thereof as oxidation dye precursors for the production of oxidation dyes.

2. The use claimed in claim 1, characterized in that, in formula I, the groups R¹, R², R³, R⁴ and R⁵ are hydrogen or, optionally, one of the groups R¹, R² and R³ is a methyl group and the others are hydrogen.

3. The use claimed in claim 1 or 2, characterized in that the indolines corresponding to formula I or salts thereof are used as color modifiers in oxidation hair dyes containing typical developer compounds.

4. Hair dyes containing oxidation dye precursors in a carrier, characterized in that indolines of formula I according to claim 1 or 2 or salts thereof are present in a quantity of 0.1 to 20 millimol per 100 g hair dye as oxidation dye precursors while a gel containing 1 to 20% by weight of a soap or an oil-in-water emulsion containing 1 to 25% by weight of a fatty component and 0.5 to 30% by weight of an emulsifier from the group of anionic, nonionic, cationic or ampholytic surfactants is present as the carrier.

5. Hair dyes as claimed in claim 4, characterized in that they contain indolines of formula I according to claim 1 or 2 or salts thereof as color modifiers and the developer compounds typically used for oxidation hair dyes.

## Revendications

1. Utilisation d'indolines de la formule I 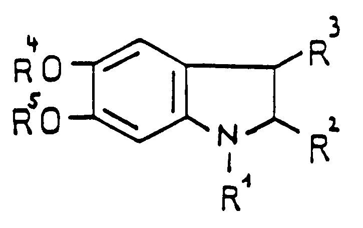 dans laquelle R¹, R², R³, R⁴ et R⁵ représentent indépendamment les uns des autres l'hydrogène ou des groupes alkyle comportant 1 a 4 atomes de C, ou R⁴ ou R⁵ correspondent, conjointement avec les atomes d'oxygène auxquels ils sont liés, à un groupe alkylènedioxy possédant 1 à 4 atomes de C, ou leurs sels comme précurseurs de colorants d'oxydation pour la fabrication de teintures d'oxydation.

2. Utilisation selon la revendication 1, caractérisée en ce que dans la formule I, les groupes R¹, R², R³, R⁴ et R⁵ représentent l'hydrogène ou en ce que, le cas échéant, un des groupes R¹, R² et R³ est un groupe méthyle et les autres sont l'hydrogène.

3. Utilisation selon la revendication 1 ou 2, caractérisée en ce que les indolines de la formule I ou leurs sels sont contenus comme composants copulateurs dans des teintures d'oxydation renfermant des composés développeurs usuels.

4. Teintures pour les cheveux contenant des précurseurs de colorants d'oxydation dans un véhicule, caractérisées en ce qu'elles renferment comme précurseurs de colorants d'oxydation, des indolines de la formule I ou leurs sels dans une proportion de 0,1 à 20 millimoles par 100 g de la teinture pour les cheveux et comme véhicule, un gel renfermant 1 à 20 % d'un savon ou une émulsion huile dans l'eau contenant 1 à 25 % en poids d'un composant gras et 0,5 à 30 % en poids d'un émulsifiant faisant partie du groupe des tensioactifs anioniques, non ioniques, cationiques ou ampholytes.

5. Teintures pour les cheveux selon la revendication 4, caractérisées en ce qu'elles renferment comme composants copulateurs, des indolines de la formule I selon la revendication 1 ou 2, ou leurs sels, ainsi que des composés développeurs usuels pour les teintures d'oxydation pour les cheveux.
